# EUROPEAN PATENT SPECIFICATION

(11) **EP 3 050 504 B1**
(45) Date of publication and mention of the grant of the patent: **17.06.2020**
(21) Application number: 14901818.6
(22) Date of filing: 22.10.2014
(51) Int. Cl.: A61B 5/1473, A61B 5/00, A61B 5/145, A61B 5/1486

(54) **CONTINUOUS GLUCOSE MONITORING SYSTEM AND MONITORING TERMINAL**
SYSTEM ZUR KONTINUIERLICHEN BLUTZUCKERÜBERWACHUNG UND ÜBERWACHUNGSENDGERÄT
SYSTÈME DE SURVEILLANCE CONTINUE DE GLUCOSE ET TERMINAL DE SURVEILLANCE

(43) Date of publication of application: 03.08.2016
(73) Proprietor: Glutalor Medical Inc, Exton, PA 19341 (US)
(72) Inventor: YU, Dongfang, Shenzhen Guangdong 518000 (CN)
(74) Representative: Proi World Intellectual Property GmbH
(86) International application number: PCT/CN2014/089184
(87) International publication number: WO 2016/061767

(56) References cited:
- CN-A- 1 596 826
- CN-A- 101 467 878
- CN-A- 102 217 939
- CN-A- 104 055 525
- US-A1- 2006 258 959
- US-A1- 2011 152 634
- US-A1- 2012 116 190
- US-A1- 2013 184 547
- US-A1- 2014 266 776
- US-B1- 7 048 687

## Description

### TECHNICAL FIELD

The present application relates to medical electronic technology field, and more particularly, relates to a continuous glucose monitoring system and a monitoring terminal.

### BACKGROUND

For a diabetic, the monitoring of glucose is very important. By monitoring the glucose, it can be determine at what time the insulin should be injected in order to lower the glucose level in human body, or to supplement the glucose in order to make the glucose reach to a normal level.

At present, generally, a mainstream domestic portable blood glucose meter available in the market (for example, the products produced by companies such as Sanrupid, Omron, Yuwel, ACCU-CHEK, or the like) uses a blood sampling method which collects the peripheral blood of a human body for the detection of the blood glucose: firstly the subcutaneous tissue liquid of a human body of a user is collected using lancing devices or blood taking papers; then the blood glucose level is detected and determined by using a colorimetric method, an electrochemical method, or a photometer. However, when using the lancing device or blood taking papers to dynamically monitor the change of the blood glucose of a user, at least four blood glucose papers are needed every day, and the skin of the user needs to be pierced at least four times. In this way, the user may repeatedly feel stabbing pains, and the user experience is poor. Besides, the information about the blood glucose acquired by dynamically monitoring the blood glucose of the user by means of the paper is very limited, and it is impossible to analyze and determine the change of the blood glucose of the user with little blood glucose information.

Besides, it is possible to detect the glucose using an electrochemical sensor. In this case, the sensor is directly implanted into the blood vessels or the subcutaneous tissues of a patient. However, in general, these apparatuses are expensive, heavy and inflexible, and have larger volumes. Besides, the detection of the glucose using an electrochemical sensor must be carried out in a hospital or an office of a doctor, which greatly limits the activities of the patient.

It is also possible to use some apparatuses to detect the glucose with a sensor guiding object which is placed on the skin of the patient or the position near the skin. In this case, the sensor guiding object of this type may be bound on the body of the patient. However, the sensor guiding object of this type is usually heavy, and can not be moved freely. Furthermore, the sensor guiding object or the sensor includes cables or wires which are configured to connect the sensor to other apparatuses for the purpose of transmitting signals from the sensor to an analyzer. The size of the sensor guiding object and the present of the cables and wires also limit the activities of the patient.

US 2012/116190 A1 discloses a continuous analysis apparatus having a sensing unit with a sensor needle for positioning in subcutaneous tissue, and a data holding unit having a storage means for storing the information obtained from the sensor or data corresponding to the information. The data holding unit is detachable from the sensing unit.

US 2013/184547 A1 discloses a glucose monitoring system having a sensor, a data processing unit connectable to the sensor, and a primary receiver unit which is configured to communicate with the data processing unit via a communication link. The sensor and the data processing unit may be configured as a single integrated assembly, or the integrated sensor and sensor electronics assembly may be configured as an on-body patch device. The on-body patch device may be configured for RFID or RF communication with a reader device/receiver unit.

### BRIEF SUMMARY

The object of the present application is to provide a continuous glucose monitoring system and a monitoring terminal, aiming at the defects in the art that the monitoring of the blood glucose is fussy, and limits the activities of the patient.

The invention aims at solving the above mentioned technical problem and it is defined by appended claims 1-10. Exemplary embodiments are described below.

In one aspect, a continuous glucose monitoring system is provided, which comprises: a monitoring terminal and a mobile terminal, wherein the monitoring terminal includes a portable host and a probe assembly which is assembled to the host; the mobile terminal includes a second data communication unit and a user interface; the probe assembly includes two glucolase micro electrode needles, a micro processor, and two electrode terminals; the host includes a signal sampling unit, a signal processing unit, a data storage unit, and a first data communication unit; wherein the signal sampling unit is configured to continuously acquire voltage sampling signals from the two electrode terminals of the probe assembly; the signal processing unit is configured to continuously generate detection data based on the voltage sampling signals, and store the detection data into the data storage unit; the first data communication unit and the second data communication unit are configured to transmit the detection data between the host and the mobile terminal in real time; and the user interface is configured to display the detection date acquired from the host.

In one exemplary embodiment, the host further includes an upper computer authentication unit which is configured to authenticate the mobile terminal before data communication is carried out between the host and the mobile terminal via the first data communication unit and the second data communication unit; after it is successfully authenticated in the upper computer authentication unit, the first data communication unit periodically sends the detection data to the second data communication unit.

In another exemplary embodiment, each probe assembly has a unique serial number, and the host further includes a probe authentication unit; the probe authentication unit is configured to read the serial number of the probe assembly which is assembled to the host, and to initiate the signal sampling unit, the signal processing unit, the data storage unit, and the first data communication unit when the serial number of the probe assembly is valid.

In a further exemplary embodiment, the system further includes a server which is connected to the mobile terminal via a wireless route; all valid serial numbers of the valid probe assemblies are stored in the server; the mobile terminal writes all valid serial numbers of the probe assemblies acquired from the server into the host via the second data communication unit.

In a further exemplary embodiment, the mobile terminal further includes a data analyzing unit, and the data analyzing unit is configured to compare the detection data acquired from the host with blood glucose reference data, give an alarm when the detection data is exceptional, acquire all detection data in the data storage unit from the host via the second data communication unit based on instructions inputted in the user interface, and display the detection data on the user interface.

In another aspect, a continuous glucose monitoring terminal is further provided, which comprises a portable host and probe assembly which is assembled to the host, wherein the probe assembly includes two glucolase micro electrode needles, a micro processor, and two electrode terminals; the host includes a signal sampling unit, a signal processing unit, a data storage unit, and a first data communication unit; wherein the signal sampling unit is configured to continuously acquire voltage sampling signals from the two electrode terminals of the probe assembly; the signal processing unit is configured to continuously generate detection data based on the voltage sampling signals, and store the detection data into the data storage unit; the first data communication unit is configured to wirelessly send the detection data at regular time intervals.

In one exemplary embodiment, each probe assembly has a unique serial number, and the host further includes a probe authentication unit; the probe authentication unit is configured to read the serial number of the probe assembly which is assembled to the host, and to initiate the signal sampling unit, the signal processing unit, and the first data communication unit when the serial number of the probe assembly is valid.

In another exemplary embodiment, the signal processing unit creates the detection data based on an average of a plurality of voltage sampling signals in a preset period of time.

In a further exemplary embodiment, the host further includes a polarizing operation controlling unit which is configured to output voltage signals to the electrode terminals of the probe assembly in accordance with a preset program when the probe assembly is assembled to the host.

In a further exemplary embodiment, the host further includes a shutdown controlling unit which is configured to start timing after the polarizing operation of the polarizing operation controlling unit is finished, send all detection data in the data storage unit to the mobile terminal via the first data communication unit when a preset period of time has passed, and meanwhile make the single sampling unit, the signal processing unit and the first data communication data stop running.

In the continuous glucose monitoring system and a monitoring terminal according to the present application, by using the portable host equipped with the probe assembly to carry out the sampling of the blood glucose data, and sending the detection data continuously to the mobile terminal to continuously monitor the blood glucose, the collection and analysis of the blood glucose data is greatly facilitated, thereby providing a reliable basis for the diabetes treatment.

The invention is defined by appended claims 1-10.

### BRIEF DESCRIPTION OF THE DRAWINGS

Fig. 1 is a block diagram of a continuous glucose monitoring system according to an exemplary embodiment of the present application;
Fig. 2 is a block diagram of the host shown in Fig. 1 according to another exemplary embodiment of the present application; and
Fig. 3 is a block diagram of the mobile terminal shown in Fig. 1 according to another exemplary embodiment of the present application.

### DETAILED DESCRIPTION

To make the object, the technical solution and the technical advantages more clearly, the present application will be further described with reference to the accompanying drawings and exemplary embodiments in the following. It should be understood that, the exemplary embodiment described here is just for explanation, not for limitation.

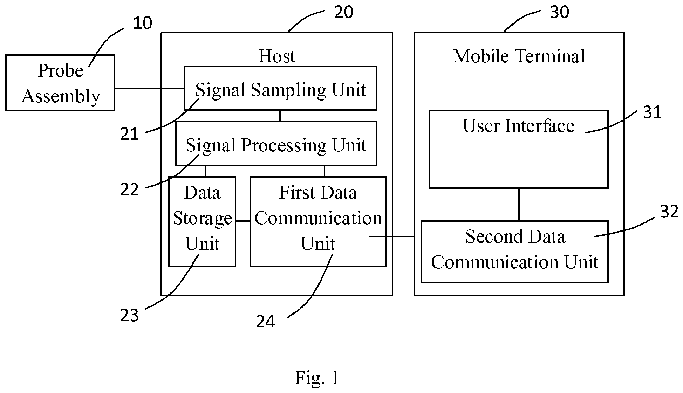

Fig.1 is a block diagram of a continuous glucose monitoring system according to an exemplary embodiment of the present application, which may continuously collect glucose data of a human body. In the present example, the continuous glucose monitoring system comprises a monitoring terminal and a mobile terminal 30, and the monitoring terminal further includes a portable host 20 and a probe assembly 10 which is assembled to the host 20. The host 20 is a reusable apparatus, and may be fixed to the human body by means of such as pasting, binding, or the like, and the host does not need to be taken off even during the exercise of the human body. However, the probe assembly 10 is a disposable product, and the probe assembly 10 is assembled to the host 20 in such a way that the probe assembly 10 is conveniently disassembled from the host 20. The mobile terminal 30 may be a cell phone, a tablet computer, a laptop or the like, which has functions of wirelessly receiving and sending data, and which has corresponding software programs installed therein.

The probe assembly 10 includes two glucolase micro electrode needles, a micro processor, and two electrode terminals. The glucolase micro electrode needles in this case are implantable probes having a length of approximately 4.9mm (the needles are implanted into the human body by piercing the surface cuticle and epidermis, and further reaching to the corium layer; for example, an implantation length of the needles into the human body may be approximately 4-4.5mm). During the oxidation reaction between the interstitial fluids of the user and the glucose inside the human body, voltage signals are generated with the processing of the micro processor, and the voltage signals are further outputted via the electrode terminal. The glucolase micro electrode needles may keep the accuracy of the detection value in seven days.

The host 20 includes a signal sampling unit 21, a signal processing unit 22, a data storage unit 23, and a first data communication unit 24. The mobile terminal 30 includes a second data communication unit 32 and a user interface 31.

The signal sampling unit 21 is configured to continuously acquire voltage sampling signals from the two electrode terminals of the probe assembly (including the process of filtering, amplifying the voltage signals, or the like). Specifically, the signal sampling unit 21 samples once every 11.25 seconds. The signal processing unit 22 is configured to continuously generate blood glucose detection data based on the voltage sampling signals, send the detection data via the first data communication unit 24, and meanwhile store the detection data in the data storage unit 23. The first data communication unit 24 and the second first data communication unit 32 are configured to transmit the detection data between the host 20 and the mobile terminal 30 in real time. For example, every time the signal processing unit 22 generates one blood glucose detection datum, the detection datum is then transmitted to the mobile terminal 30 via the first data communication unit 24 and the second first data communication unit 32. The user interface 31 of the mobile terminal 30 is configured to display the detection date acquired from the host 20, in such a way that the user may learn about the blood glucose data.

In particular, it is possible for the first data communication unit 24 and the second first data communication unit 32 to be a Bluetooth communication module, and transmit data via Bluetooth.

By the glucolase micro electrode needles implanted into the epidermis, and combing with the host and the mobile terminal, the continuous glucose monitoring system may continuously collect the blood glucose data of the human body. Compared with the prior art, the continuous glucose monitoring system is convenient, brings about a slight pain, and is bloodless, which will not only improve the blood glucose monitoring experience of the user, but also greatly reduce the detection cost when a same number of times of monitoring are carried out. In this way, it is possible to continuously acquire a large number of blood glucose values for a long time.

The signal processing unit 22 of the host 20 creates the detection data based on the average of a plurality of voltage sampling signals in a preset period of time; for example, the signal sampling unit 21 samples once every 11.25 seconds, and the signal processing unit 22 generates a blood glucose detection datum every three minutes, that is, the signal processing unit 22 creates a blood glucose detection datum based on the average of sixteen voltage sampling signals sampled by the signal sampling unit 21 in three minutes. In this way, the accuracy of the blood glucose detection data may be ensured, and the blood glucose detection data are avoided from being influenced due to the fluctuation of the voltage sampling data.

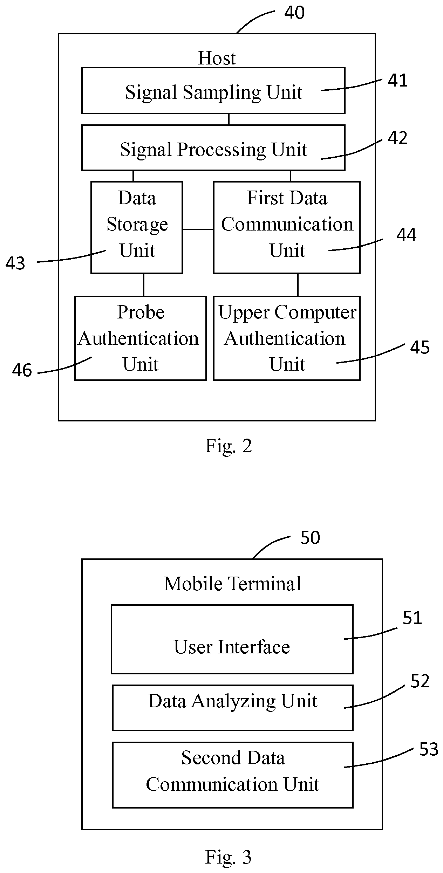

Fig. 2 is a block diagram showing another exemplary embodiment of the host of the continuous glucose monitoring system. The host 40 in this example includes a signal sampling unit 41, a signal processing unit 42, a data storage unit 43, and a first data communication unit 44; besides, the host 40 further includes a probe authentication unit 46.

In this exemplary embodiment, each probe assembly 10 has a unique serial number (for example, the serial number may be stored in the micro processor of the probe assembly 10). The probe authentication unit 46 of the host 40 is configured to read the serial number of the probe assembly which is assembled to the host 40, and initiate the signal sampling unit 41, the signal processing unit 42, the data storage unit 43, and the first data communication unit 44 when the serial number of the probe assembly is valid. Thereby, the processes, such as sampling the voltage signals, and converting the voltage sampling signals into the blood glucose detection data, are taken place.

The serial number of the probe assembly may be stored in a remote server which is connected to the mobile terminal 30 via a wireless route. All valid serial numbers of the probe assembly are stored in the server, and the mobile terminal 30 writes all valid serial numbers of the probe assemblies acquired from the server into the host 40 via the second data communication unit 32.

In particular, since the probe assembly 10 is a disposable product, once the probe assembly 10 is activated, the host 40 sends the serial number of the probe assembly 10 to the remote server via the mobile terminal 30, and the server further sets the serial number as invalid.

By the serial number authentication between the host 40 and the probe assembly 10, it can be ensure that the probe assembly 10 is valid, preventing the blood glucose detection data from getting wrong due to the usage of inferior products, and further preventing the probe assembly from being repeatedly used; in this way, the cross infection and the reduction of detection accuracy of the blood glucose may be avoided.

The host 40 may further includes an upper computer authentication unit 45 which is configured to authenticate the mobile terminal 30, before data communication is carried out between the host 40 and the mobile terminal 30 via the first data communication unit 44 and the second data communication unit 32. After it is successfully authenticated in the upper computer authentication unit 45, the first data communication unit 44 periodically sends the detection data to the second data communication unit 32. In specific, it is possible for the upper computer authentication unit 45 to authenticate the mobile terminal 30 by means of password. That is, it is confirmed that it is successfully authenticated only when the password inputted in the mobile terminal 30 is correct, and only at this time, the host 40 will send the blood glucose detection data to the mobile terminal 30, or receive the control instructions.

The host 40 may further include a polarizing operation controlling unit (not shown in Fig. 2). The polarizing operation controlling unit is configured to activate the probe assembly 10. In specific, the polarizing operation controlling unit outputs voltage signals to the electrode terminals of the probe assembly 10 in accordance with a preset program, when the probe assembly 10 is assembled to the host 40.

Furthermore, the host 40 may further include a shutdown controlling unit which is configured to start timing after the polarizing operation of the polarizing operation controlling unit is finished, send all detection data in the data storage unit 43 to the mobile terminal 30 via the first data communication unit 44 when a preset period of time (such as seven days) has passed, and meanwhile make the single sampling unit 41, the signal processing unit 42 and the first data communication data 44 stop running. After the shutdown controlling unit shuts down the host, the user may again use the host 40 to carry out continuous glucose monitoring when the probe assembly has been changed.

Fig. 3 is a block diagram of the mobile terminal in the continuous glucose monitoring system according to another exemplary embodiment. In addition to a user interface 51 and a second data communication unit 53, the mobile terminal 50 further includes a data analyzing unit 52. The data analyzing unit 52 is configured to compare the detection data acquired from the host 20 with blood glucose reference data, give an alarm when the detection data is exceptional (for example, to send a reminder of injecting insulin, supplementing blood glucose, or the like), acquire all detection data in the data storage unit 23 from the host 20 via the second data communication unit 53 based on the instructions inputted in the user interface 51, and display the detection data on the user interface 51 (for example, in form of a blood glucose dynamic change graph or a change curve).

By means of the data analyzing unit 52, the mobile terminal draws a blood glucose dynamic change graph based on the blood glucose detection data within a given period of time, thus assisting to determine the blood glucose state of the user. Besides, it is possible for the data analyzing unit 52 to periodically upload the blood glucose value and the blood glucose dynamic change graph of the user to a health network cloud, which provides a reliable basis for the diagnosis of a doctor or an expert.

The invention is defined by appended claims 1 -10.

## Claims

1. A continuous glucose monitoring terminal, comprising a portable host (20, 40) and probe assembly (10) wherein the probe assembly (10) includes two glucolase micro electrode needles, a micro processor, and two electrode terminals;
the host (20, 40) includes a signal sampling unit (21, 41), a signal processing unit (22, 42), a data storage unit (23, 43), and a first data communication unit (24, 44); wherein the signal sampling unit (21, 41) is configured to continuously acquire voltage sampling signals from the two electrode terminals of the probe assembly (10); the signal processing unit (22, 42) is configured to continuously generate detection data based on the voltage sampling signals, and store the detection data into the data storage unit (23, 43); the first data communication unit (24, 44) is configured to wirelessly send the detection data at regular time intervals to a mobile terminal (30), the host (20, 40) is a reusable apparatus, and is configured to be fixed to the human body by means of pasting or binding; the probe assembly (10) is a disposable product, and the probe assembly (10) is configured to be assembled to the host (20, 40) during operation of the continuous glucose monitoring terminal and to be disassembled from the host (20, 40) afterwards.

2. The continuous glucose monitoring terminal according to claim 1, wherein each probe assembly (10) has a unique serial number, and the host (40) further includes a probe authentication unit (46); the probe authentication unit (46) is configured to read the serial number of the probe assembly (10) which is assembled to the host (40), and to initiate the signal sampling unit (41), the signal processing unit (42), and the first data communication unit (44) when the serial number of the probe assembly (10) is valid.

3. The continuous glucose monitoring terminal according to claim 1, wherein the signal processing unit (22, 42) creates the detection data based on an average of a plurality of voltage sampling signals in a preset period of time.

4. The continuous glucose monitoring terminal according to claim 1, wherein the host (20, 40) further includes a polarizing operation controlling unit which is configured to output voltage signals to the electrode terminals of the probe assembly (10) in accordance with a preset program when the probe assembly (10) is assembled to the host (20, 40).

5. The continuous glucose monitoring terminal according to claim 4, wherein the host (20, 40) further includes a shutdown controlling unit which is configured to start timing after the polarizing operation of the polarizing operation controlling unit is finished, send all detection data in the data storage unit (23, 43) to the mobile terminal (30, 50) via the first data communication unit (24, 44) when a preset period of time has passed, and meanwhile make the single sampling unit, the signal processing unit and the first data communication data stop running.

6. A continuous glucose monitoring system, comprising: a mobile terminal (30, 50) and a continuous glucose monitoring terminal according to one of claims 1-5.

7. The continuous glucose monitoring system according to claim 6, wherein the host (40) further includes an upper computer authentication unit (45) which is configured to authenticate the mobile terminal (50) before data communication is carried out between the host (20) and the mobile terminal (50) via the first data communication unit (24, 44) and the second data communication unit (32, 53); after it is successfully authenticated in the upper computer authentication unit (45), the first data communication unit (24, 44) is configured to periodically send the detection data to the second data communication unit (32, 53).

8. The continuous glucose monitoring system according to claim 6, wherein each probe assembly (10) has a unique serial number, and the host (20) further includes a probe authentication unit (46); the probe authentication unit (46) is configured to read the serial number of the probe assembly (10) which is assembled to the host (20), and to initiate the signal sampling unit, the signal processing unit, the data storage unit, and the first data communication unit when the serial number of the probe assembly (10) is valid.

9. The continuous glucose monitoring system according to claim 8, wherein the system further includes a server which is connected to the mobile terminal (30, 50) via a wireless route; all valid serial numbers of the valid probe assemblies are stored in the server; the mobile terminal (30, 50) writes all valid serial numbers of the probe assemblies acquired from the server into the host (20) via the second data communication unit (53).

10. The continuous glucose monitoring system according to claim 6, wherein the mobile terminal (50) further includes a data analyzing unit (52), and the data analyzing unit (52) is configured to compare the detection data acquired from the host (20) with blood glucose reference data, give an alarm when the detection data is exceptional, acquire all detection data in the data storage unit (23, 43) from the host (20) via the second data communication unit (53) based on instructions inputted in the user interface, and display the detection data on the user interface.

## Patentansprüche

1. Kontinuierliches Glukose-Überwachungs-Terminal, das einen tragbaren Host (20, 40) und eine Sondenanordnung (10) umfasst, wobei
die Sondenanordnung (10) zwei Glukolase-Mikroelektrodennadeln, einen Mikroprozessor und zwei Elektrodenanschlüsse umfasst;
wobei der Host (20, 40) eine Signalabtasteinheit (21, 41), eine Signalverarbeitungseinheit (22, 42), eine Datenspeichereinheit (23, 43) und eine erste Datenkommunikationseinheit (24, 44) aufweist;
wobei die Signalabtasteinheit (21, 41) so konfiguriert ist, dass sie kontinuierlich Spannungsabtastsignale von den beiden Elektrodenanschlüssen der Sondenanordnung (10) erfasst;
die Signalverarbeitungseinheit (22, 42) so konfiguriert ist, dass sie kontinuierlich Erfassungsdaten auf der Grundlage der Spannungsabtastsignale erzeugt und die Erfassungsdaten in der Datenspeichereinheit (23, 43) speichert;
die erste Datenkommunikationseinheit (24, 44) so konfiguriert ist, dass sie die Erfassungsdaten in regelmäßigen Zeitintervallen drahtlos an ein mobiles Terminal (30) sendet,
der Host (20, 40) eine wiederverwendbare Vorrichtung ist und so konfiguriert ist, dass er am menschlichen Körper durch Kleben oder Anbinden befestigt werden kann;
die Sondenanordnung (10) ein Einwegprodukt ist und die Sondenanordnung (10) so konfiguriert ist, dass sie während des Betriebs des kontinuierlichen Glukose-Überwachungsterminals an den Host (20, 40) montiert und anschließend vom Host (20, 40) abgebaut werden kann.

2. Kontinuierliches Glukose-Überwachungs-Terminal nach Anspruch 1, wobei jede Sondenanordnung (10) eine eindeutige Seriennummer hat, und der Host (40) umfasst ferner eine Sonden-Authentifizierungseinheit (46); die Sonden-Authentifizierungseinheit (46) ist konfiguriert, um die Seriennummer der an den Host (40) montierten Sondenanordnung (10) zu lesen und die Signalabtasteinheit (41), die Signalverarbeitungseinheit (42) und die erste Datenkommunikationseinheit (44) zu initiieren, wenn die Seriennummer der Sondenanordnung (10) gültig ist.

3. Kontinuierliches Glukose-Überwachungs-Terminal nach Anspruch 1, wobei die Signalverarbeitungseinheit (22, 42) die Erfassungsdaten auf der Grundlage eines Mittelwertes einer Vielzahl von Spannungsabtastsignalen in einer vorgegebenen Zeitperiode erzeugt.

4. Kontinuierliches Glukose-Überwachungs-Terminal nach Anspruch 1, wobei der Host (20, 40) ferner eine Polarisationsbetriebssteuereinheit umfasst, die so konfiguriert ist, dass sie Spannungssignale an die Elektrodenanschlüsse der Sondenanordnung (10) gemäß einem voreingestellten Programm ausgibt, wenn die Sondenanordnung (10) an den Host (20, 40) montiert ist.

5. Kontinuierliches Glukose-Überwachungs-Terminal nach Anspruch 4, wobei der Host (20, 40) ferner eine Abschalt-Steuereinheit umfasst, die so konfiguriert ist, dass sie die Zeitmessung beginnt, nachdem der Polarisationsvorgang der Polarisationsbetriebssteuereinheit beendet ist, dass sie alle Erfassungsdaten in der Datenspeichereinheit (23, 43) über die erste Datenkommunikationseinheit (24, 44) an das mobile Endgerät (30, 50) sendet, wenn eine voreingestellte Zeitspanne verstrichen ist, und dass sie in der Zwischenzeit die einzelne Abtasteinheit, die Signalverarbeitungseinheit und die ersten Datenkommunikationseinheit zum Anhalten des Betriebs veranlasst.

6. Kontinuierliches Glukose-Überwachungssystem, umfassend: ein mobiles Terminal (30, 50) und ein kontinuierliches Glukose-Überwachungs-Terminal nach einem der Ansprüche 1-5.

7. Kontinuierliches Glukose-Überwachungssystem nach Anspruch 6, wobei der Host (40) ferner eine obere Computer-Authentifizierungseinheit (45) umfasst, die so konfiguriert ist, dass sie das mobile Endgerät (50) authentifiziert, bevor die Datenkommunikation zwischen dem Host (20) und dem mobilen Endgerät (50) über die erste Datenkommunikationseinheit (24, 44) und die zweite Datenkommunikationseinheit (32, 53) ausgeführt wird; nachdem sie in der oberen Computer-Authentifizierungseinheit (45) erfolgreich authentifiziert wurde, ist die erste Datenkommunikationseinheit (24, 44) so konfiguriert, dass sie die Erkennungsdaten periodisch an die zweite Datenkommunikationseinheit (32, 53) sendet.

8. Kontinuierliches Glukose-Überwachungssystem nach Anspruch 6, wobei jede Sondenanordnung (10) eine eindeutige Seriennummer hat und der Host (20) ferner eine Sonden-Authentifizierungseinheit (46) aufweist; die Sonden-Authentifizierungseinheit (46) ist konfiguriert, die Seriennummer der Sondenanordnung (10) zu lesen, die mit dem Host (20) zusammengebaut ist, und die Signalabtasteinheit, die Signalverarbeitungseinheit, die Datenspeicherungseinheit und die erste Datenkommunikationseinheit zu initiieren, wenn die Seriennummer der Sondenanordnung (10) gültig ist.

9. Kontinuierliches Glukose-Überwachungssystem nach Anspruch 8, wobei das System ferner einen Server umfasst, der über eine drahtlose Route mit dem mobilen Endgerät (30, 50) verbunden ist; alle gültigen Seriennummern der gültigen Sondenanordnungen im Server gespeichert sind; das mobile Endgerät (30, 50) alle gültigen Seriennummern der vom Server erfassten Sondenanordnungen über die zweite Datenkommunikationseinheit (53) in den Host (20) schreibt.

10. Kontinuierliches Glukose-Überwachungssystem nach Anspruch 6, wobei das mobile Endgerät (50) ferner eine Datenanalyseeinheit (52) umfasst und die Datenanalyseeinheit (52) so konfiguriert ist, dass sie die von dem Host (20) erfassten Erfassungsdaten mit Blutzucker-Referenzdaten vergleicht, einen Alarm auslöst, wenn die Erfassungsdaten außergewöhnlich sind, alle Erfassungsdaten in der Datenspeichereinheit (23, 43) von dem Host (20) über die zweite Datenkommunikationseinheit (53) auf der Grundlage von in die Benutzerschnittstelle eingegebenen Anweisungen erfasst und die Erfassungsdaten auf der Benutzerschnittstelle anzeigt.

## Revendications

1. Terminal de surveillance continue du glucose, comprenant un hôte portatif (20, 40) et un ensemble sonde (10) dans lequel
l'ensemble sonde (10) comporte deux aiguilles de microélectrode de glucolase, un microprocesseur et deux bornes pour électrode ;
l'hôte (20, 40) comporte une unité d'échantillonnage du signal (21, 41), une unité de traitement du signal (22, 42), une unité de stockage des données (23, 43) et une première unité de communication des données (24, 44); dans lequel l'unité d'échantillonnage du signal (21, 41) est configurée pour acquérir en continu des signaux d'échantillonnage de tension à partir des deux terminaux d'électrode de l'ensemble sonde (10) ; l'unité de traitement du signal (22, 42) est configurée pour générer en continu des données de détection basées sur les signaux d'échantillonnage de tension et mémorise les données de détection dans l'unité de stockage des données (23, 43) ; la première unité de communication des données (24, 44) est configurée pour envoyer sans fil les données de détection à intervalles réguliers vers un terminal mobile (30),
l'hôte (20, 40) est un appareil réutilisable et est configuré pour être fixé au corps humain au moyen de collage ou de liaison ; l'ensemble sonde (10) est un produit jetable et l'ensemble sonde (10) est configuré pour être assemblé à l'hôte (20, 40) pendant le fonctionnement du terminal de surveillance continue du glucose 11 Z et être désassemblé de l'hôte (20, 40) par la suite.

2. Terminal de surveillance continue du glucose selon la revendication 1, dans lequel chaque ensemble sonde (10) possède un numéro de série unique et l'hôte (40) inclut comporte en outre une unité d'authentification de la sonde (46) ; l'unité d'authentification de la sonde (46) est configurée pour lire le numéro de série de l'ensemble sonde (10) qui est assemblé sur l'hôte (40) et pour déclencher l'unité d'échantillonnage du signal (41), l'unité de traitement du signal (42) et la première unité de communication des données (44) lorsque le numéro de série de l'ensemble sonde (10) est valide.

3. Terminal de surveillance continue du glucose selon la revendication 1, dans lequel l'unité de traitement du signal (22, 42) crée les données de détection basées sur une moyenne d'une pluralité de signaux d'échantillonnage de tension dans une période prédéfinie.

4. Terminal de surveillance continue du glucose selon la revendication 1, dans lequel l'hôte (20, 40) comporte en outre une unité de commande de mise en œuvre de la polarisation qui est configurée pour délivrer des signaux de tension de sortie aux bornes de l'électrode de l'ensemble sonde (10) en fonction d'un programme prédéfini lorsque l'ensemble sonde (10) est assemblé à l'hôte (20, 40).

5. Terminal de surveillance continue du glucose selon la revendication 4, dans lequel l'hôte (20, 40) comporte en outre une unité de commande d'arrêt qui est configurée pour démarrer la synchronisation après que la mise en œuvre de la polarisation de l'unité de commande de mise en œuvre de la polarisation est terminée, envoie toutes les données de détection dans l'unité de stockage des données (23, 43) vers le terminal mobile (30, 50) par l'intermédiaire de la première unité de communication des données (24, 44) lorsqu'une période prédéfinie est écoulée et pendant ce temps, fait que l'unité d'échantillonnage unique, l'unité de traitement du signal et les premières données de communication de données cessent de fonctionner.

6. Système de surveillance continue du glucose, comprenant : un terminal mobile (30, 50) et un terminal de surveillance continue du glucose selon l'une des revendications 1 à 5.

7. Système de surveillance continue du glucose selon la revendication 6, dans lequel l'hôte (40) comporte en outre une unité d'authentification de l'ordinateur supérieur (45) configurée pour authentifier le terminal mobile (50) avant que la communication de données soit effectuée entre l'hôte (20) et le terminal mobile (50) par l'intermédiaire de la première unité de communication des données (24, 44) et la seconde unité de communication des données (32, 53) ; après que l'authentification est réussie dans l'unité supérieure d'authentification de l'ordinateur (45), la première unité de communication des données (24, 44) est configurée pour envoyer périodiquement les données de détection à la seconde unité de communication des données (32, 53).

8. Système de surveillance continue du glucose selon la revendication 6, dans lequel chaque ensemble sonde (10) possède un numéro de série unique et l'hôte (20) inclut comporte en outre une unité d'authentification de la sonde (46) ; l'unité d'authentification de la sonde (46) est configurée pour lire le numéro de série de l'ensemble sonde (10) qui est assemblé sur l'hôte (20) et pour déclencher l'unité d'échantillonnage du signal, l'unité de traitement du signal, l'unité de stockage des données et la première unité de communication des données lorsque le numéro de série de l'ensemble sonde (10) est valide.

9. Système de surveillance continue du glucose selon la revendication 8, dans lequel le système comporte en outre un serveur qui est connecté au terminal mobile (30, 50) par l'intermédiaire d'une voie sans fil ; tous les numéros de série valides des ensembles sondes valides sont mémorisés dans le serveur ; le terminal mobile (30, 50) écrit tous les numéros de série valides des ensembles sondes acquis depuis le serveur dans l'hôte (20) par l'intermédiaire de la seconde unité de communication des données (53).

10. Système de surveillance continue du glucose selon la revendication 6, dans lequel le terminal mobile (50) comporte en outre une unité d'analyse des données (52) et l'unité d'analyse des données (52) est configurée pour comparer les données de détection acquises à partir de l'hôte (20) avec les données de référence de la glycémie, déclencher une alarme lorsque les données de détection sont exceptionnelles, acquérir toutes les données de détection dans l'unité de stockage des données (23, 43) depuis l'hôte (20) par l'intermédiaire de la seconde unité de communication des données (53) basées sur les instructions saisies dans l'interface utilisateur et afficher les données de détection sur l'interface utilisateur.
